# EUROPÄISCHE PATENTSCHRIFT

(11) **EP 0 355 136 B1**
(45) Veröffentlichungstag und Bekanntmachung des Hinweises auf die Patenterteilung: **13.07.1994**
(21) Anmeldenummer: 89900578.9
(22) Anmeldetag: 22.12.1988
(51) Int. Cl.: A61K 6/06

(54) **ZAHNFÜLLUNGSMATERIAL**
DENTAL FILLING MATERIAL
MATIERE D'OBTURATION DENTAIRE

(30) Priorität: 23.12.1987 DE 3743719
(43) Veröffentlichungstag der Anmeldung: 28.02.1990
(73) Patentinhaber: BEGO Bremer Goldschlägerei Wilh. Herbst GmbH & Co., 28329 Bremen (DE)
(72) Erfinder: Wiedemann, Wolfgang, Dr., W-97204 Höchberg (DE)
(74) Vertreter: Möller, Friedrich, Dipl.-Ing.
(86) Internationale Anmeldenummer: DE8800777
(87) Internationale Veröffentlichungsnummer: WO8905625

(56) Entgegenhaltungen:
- EP-A- 0 230 570
- WO-A-86/01726
- DE-A- 2 410 084
- DE-A- 3 531 144
- US-A- 4 518 430

## Beschreibung

Die Erfindung betrifft ein Zahnfüllungsmaterial zum Einbringen in Kavitäten in der Hartsubstanz eines natürlichen Zahnes.

Bei bestimmten Erkrankungen der Zähne - eine der diesbezüglich bekanntesten ist die Karies - wird der Zahnschmelz und teilweise das darunter befindliche Dentin angegriffen und zerstört. Als präparative Maßnahme zur Beseitigung der Schäden und Bewahrung des Zahnes werden durch den Zahnarzt folgende Maßnahmen ergriffen: Zunächst wird der beschädigte Bereich von Zahnschmelz und Dentin mit Hilfe eines Bohrers entfernt, so daß die Kavität entsteht. Dann erfolgt eine Desinfektion und ein Ausfüllen dieser Kavität. Das hierfür eingesetzte Material soll möglichst undurchlässig sein, um Eindringen und Festsetzen von Bakterien, Schmutz oder ähnlichem zu verhindern. In der Praxis werden hierfür als Materialien Amalgam, Kunststoff, Zement, Gold oder dergleichen eingesetzt. Zum Schluß wird die Oberfläche poliert und insbesondere im Bereich der für erneute Entstehung von Karies besonders anfälligen Übergangszonen zwischen Zahnfüllmaterial und gesundem Zahn geglättet.

Zahlreiche wissenschaftliche Veröffentlichungen belegen, daß für die Wahl des Zahnfüllmaterials bislang neben der guten Handhabbarkeit in erster Linie der Gesichtspunkt möglichst hoher Undurchlässigkeit und biologisch-chemischer Inaktivität entscheidend ist.

Aus der EP-A-0 230 570 und der DE-A-35 31 144 ist Hydroxylapatit als Knochen- bzw. Zahnwurzelersatz bekannt. Die Eignung dieses Hydroxylapatits für ein Zahnfüllungsmaterial zum Einbringen in Kavitäten in der Hartsubstanz eines natürlichen Zahnes geht aus diesem Stand der Technik allerdings nicht hervor.

Der Erfindung liegt die Aufgabe zugrunde, ein neuartiges Zahnfüllungsmaterial zu schaffen, bei dem zur Heilung bestimmter Zahnerkrankungen die in der Mundhöhle befindlichen chemischen und biologischen Substanzen genutzt werden.

Zur Lösung dieser Aufgabe weist das erfindungsgemäße Zahnfüllungsmaterial poröse Calcium-Phosphatverbindungen auf, die einen Porenanteil von 20 - 70 Vol.-% aufweisen. Die Erfindung geht davon aus, daß im Speichel der Mundhöhle sich Calcium- und Phosphat-Ionen befinden, die in wechselnden, u.a. durch die momentan aufgenommene Nahrung bestimmten Anteilen vorhanden sind. Darauf aufbauend besteht der Kerngedanke der Erfindung darin, durch die Verwendung des porösen Zahnfüllungsmaterials den sich im Speichel befindlichen Ionen die Möglichkeit zu geben, in die Kavität zu diffundieren und einzudringen und sich dort in kristalliner Struktur niederzuschlagen, wodurch festes Calciumphosphat entsteht, das in seiner chemischen Zusammensetzung als auch seiner kristallinen Struktur weitgehend mit dem natürlichen Zahnschmelz übereinstimmt, so daß auf diese Weise die Kavität sukzessive mit Calciumphosphat bzw. Zahnschmelz gefüllt wird. Die Calcium- und Phosphat-Ionen schlagen sich in kristalliner Struktur nieder und gehen mit dem Zahnfüllungsmaterial eine Verbindung ein, die im wesentlichen eine identische Struktur zum natürlichen Zahnschmelz aufweist. Das in der Kavität befindliche Zahnfüllungsmaterial wirkt dabei als dreidimensionale Struktur mit Kristallisationskeimen für die Calcium- und Phosphat-Ionen.

Die vorstehend erwähnten Eigenschaften werden im wesentlichen dadurch erzielt, daß das Material Poren aufweist, die 20 - 70 Vol.-% des gesamten Materials ausmachen. Wird der Volumenanteil der Poren geringer, wird die Diffusion der Calcium- und Phosphat-Ionen in das Material zu stark behindert und unterbunden. Steigt der Anteil der Poren über 70 Vol.-%, werden die Hohlräume zu groß und es erschwert sich die Ausbildung der kristallinen Strukturen durch die dann zu geringe Anzahl und Dichte an Kristallisationskeimen.

Die erfindungsgemäße Lehre steht im diametralen Gegensatz zur bisherigen Lehre, die möglichst hohe Undurchlässigkeit gefordert hat.

Aus der US-A-4 518 430 ist eine Füllpaste für verschiedene Anwendungen bekannt. Sie besteht allerdings nicht aus Calcium-Phosphat-Verbindungen. Soweit vorgesehen ist, die Füllpaste porös auszubilden, hat das den Zweck, die Verbindung des zur Bildung von Knochenimplantaten oder Prothesen dienenden Füllmaterials mit organischem Knochengewebe zu erleichtern oder zu ermöglichen. Ein ähnlicher Stand der Technik ergibt sich aus der W0-A-86/01726.

Aus der bereits erwähnten EP-A-0 230 570 und der DE-A-35 31 144 ist poröses Hydroxylapatit-Material bekannt, das vor allem als Knochenersatz- und Knochenfüllmaterial dient. Dieses Hydroxylapatit-Material ist deshalb porös ausgebildet, um das Anwachsen von Knochengewebe zu ermöglichen bzw. zu erleichtern. Aus diesem Stand der Technik ist es hingegen nicht bekannt, Zahnfüllungsmaterial zum Einbringen in Kavitäten porös auszubilden, damit in die Poren Calcium- und Phosphat-Ionen eindringen und sich hier in kristalliner Struktur niederschlagen können.

Die Vorgehensweise beim Einbringen des erfindungsgemäßen Zahnfüllungsmaterials in Kavitäten in der Hartsubstanz eines natürlichen Zahnes ist folgende: Zunächst wird in an sich üblicher Weise der beschädigte Bereich vom Zahnschmelz und Dentin entfernt und nach erfolgter Desinfektion die Kavität ausgefüllt. Die vom Zahnarzt vorzunehmenden Arbeiten sind damit beendet und gleichen den bisherigen. Allerdings ist entscheidend, daß der Zahn damit noch nicht "fertig" ist. Vielmehr setzt nun völlig selbsttätig eine Diffusion der im Speichel befindlichen Calcium- und Phosphat-Ionen in das Zahnfüllungsmaterial ein und bildet dort die kristalline Calcium-Phosphat-Struktur.

Die durch die Erfindung erreichbaren Vorteile sind vielfältig: So wird kein fremdes Material in die Kavität eingebracht, sondern das Verschließen erfolgt durch die gleiche chemische Substanz, nämlich Calciumphosphat, aus der auch der Zahnschmelz aufgebaut ist. Die sich ausbildende Härte im Bereich der Kavität stimmt folglich mit der des Zahnschmelzes überein. Schließlich erfolgen die Übergänge im Randbereich zwischen Kavität und Zahn durch die durch Anwachsen sich ausbildenden kristallinen Strukturen glatt und ohne Kanten.

Bei großer Tiefe der Kavität kann mitunter der Fall eintreten, daß das Zahnfüllungsmaterial seine kristallinen Strukturen zunächst an der Oberfläche ausbildet und dadurch die Kavität verschließt, so daß die Calcium- und Phosphat-Ionen nicht in ausreichendem Maße bis in die Tiefe der Kavität eindiffundieren können. Dieser Nachteil tritt vor allem dann auf, wenn die Kavität in nennenswerter Tiefe in das Dentin hineinreicht. Eine Lösung dieses Problems besteht darin, die Sohle der Kavität vor dem Einbringen des erfindungsgemäßen Zahnfüllungsmaterials mit einer Unterfüllung zu versehen.

Als Material für die Zahnfüllung werden konkret poröse Calcium-Phosphatverbindungen vorgeschlagen. Calciumphosphat ist in seiner chemischen Struktur mit der des Zahnschmelzes identisch, weist jedoch nicht die zur Ausbildung der erforderlichen Härte des Zahnschmelzes kristalline Struktur auf. Die Porösität entsteht dadurch, daß Basismaterial gleicher chemischer Beschaffenheit gemahlen und anschließend gesintert wird.

Als in besonderer Weise geeignet wird das Material Hydroxylapatit, eine basische Calcium-Phosphatverbindung, poröses Fluorapatit - diese Verbindung enthält bereits das vorteilhafte Fluor - sowie entkalkter tierischer Zahnschmelz vorgeschlagen. Ohne Beeinträchtigung der Allgemeinheit eignen sich diese Materialien zur Zahnfüllung in ganz besonderer Weise.

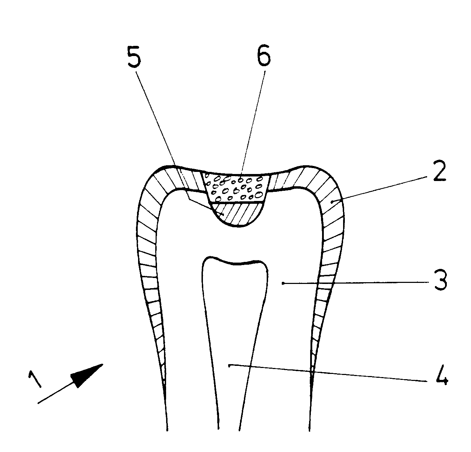

Weitere Einzelheiten, Merkmale und Vorteile der Erfindung ergeben sich aus der nachfolgenden Beschreibung, indem anhand der Zeichnung ein Ausführungsbeispiel der Erfindung in prinzipieller Darstellung wiedergegeben ist. Sie zeigt einen Querschnitt durch einen präparierten Zahn.

Der Zahn 1 ist in an sich bekannter Weise an seiner Oberfläche mit dem aufgrund seiner kristallinen Struktur harten Zahnschmelz 2 versehen. Nach innen zu schließt sich das Dentin 3 an, welches an der Pulpa 4 endet. Dieses entspricht dem Aufbau eines gesunden Zahnes.

Zur Beseitigung einer z.B. kariösen Stelle wird der Zahnschmelz und unter Umständen das darunter befindliche Dentin durch Ausbohren entfernt. Anschließend wird die Kavität durch an sich bereits bekanntes Material mit einer Unterfüllung 5 versehen. Für die Erfindung entscheidend ist, daß dann oberhalb und auf der Höhe des Zahnschmelzes 2 das poröse Zahnfüllungsmaterial eingebracht wird. Die Arbeiten wären damit abgeschlossen, die Füllung allerdings noch nicht in ihrem endgültigen Zustand, da nunmehr durch Diffusion von im Speichel befindlichen Calcium- und Phosphat-Ionen in das Innere des porösen Zahnfüllungsmaterials kristalline und dem Zahnschmelz vergleichbare Calcium-Phosphat-Strukturen entstehen.

Nach einer gewissen Zeit erhält man den bekannten Zahnfüllungsmaterialien, wie Amalgam, Gold und Kunststoff, in ihrer Verträglichkeit und auch Festigkeit weit überlegene Strukturen im Bereich der Kavität.

## Patentansprüche

1. Zahnfüllungsmaterial zum Einbringen in Kavitäten in der Hartsubstanz eines natürlichen Zahnes, **gekennzeichnet durch** poröse Calcium-Phosphatverbindungen, die einen Porenanteil von 20 bis 70 Vol.-% aufweisen.

2. Zahnfüllungsmaterial nach Anspruch 1, dadurch gekennzeichnet, daß die Calcium-Phosphatverbindungen Hydroxylapatit sind.

3. Zahnfüllungsmaterial nach Anspruch 1, dadurch gekennzeichnet, daß die Calcium-Phosphatverbindungen Fluorapatit sind.

4. Zahnfüllungsmaterial nach Anspruch 1, dadurch gekennzeichnet, daß die Calcium-Phosphatverbindungen vorzugsweise entkalkter tierischer Zahnschmelz sind.

## Claims

1. Dental filling material for introduction into cavities in the hard substance of a natural tooth, characterized by porous calcium phosphate compounds which have a pore content of 20 to 70 % by volume.

2. Dental filling material according to Claim 1, characterized in that the calcium phosphate compounds are hydroxyapatite.

3. Dental filling material according to Claim 1, characterized in that the calcium phosphate compounds are fluoroapatite.

4. Dental filling material according to Claim 1, characterized in that the calcium phosphate compounds are preferably decalcified animal tooth enamel.

## Revendications

1. Matériau de remplissage dentaire, à insérer dans des cavités de la substance dure d'une dent naturelle, caractérisé par des combinaisons calcium-phosphate poreuses, présentant une proportion de pores comprise dans la plage allant de 20 à 70 % en volume.

2. Matériau de remplissage dentaire selon la revendication 1, caractérisé en ce que les combinaisons calcium-phosphate sont de l'hydroxylapatite.

3. Matériau de remplissage dentaire selon la revendication 1, caractérisé en ce que les combinaisons calcium-phosphate sont de la fluorapatite.

4. Matériau de remplissage dentaire selon la revendication 1, caractérisé en ce que les combinaisons calcium-phosphate sont de préférence un produit fondu d'origine dentaire, animale, décalcifié.
